# EUROPÄISCHE PATENTANMELDUNG

(11) **EP 1 090 585 A1**
(43) Veröffentlichungstag der Anmeldung: **11.04.2001**
(21) Anmeldenummer: 00203392.6
(22) Anmeldetag: 27.09.2000
(51) Int. Cl.: A61B 6/00

(54) **C-Bogen-Röntgeneinrichtung**

(30) Priorität: 05.10.1999 DE 19947809
(71) Anmelder: Philips Corporate Intellectual Property GmbH, 52064 Aachen (DE); Koninklijke Philips Electronics N.V., 5621 BA Eindhoven (NL)
(72) Erfinder: Klotz, Erhard, 52064 Aachen (DE); Schomberg, Hermann, 52064 Aachen (DE)
(74) Vertreter: Volmer, Georg, Dipl.-Ing.

(57) **Zusammenfassung**

Die Erfindung betrifft eine Röntgeneinrichtung mit einem eine Röntgenquelle (2) und einen Röntgendetektor (3) tragenden C-Bogen (12) an einem Gelenk (13) tragenden Aufhängevorrichtung (14), wobei Röntgenquelle (2) und Röntgendetektor (3) um eine durch das Gelenk (13) verlaufende Propellerachse (z₄) rotierbar sind. Um bei einer solchen Röntgeneinrichtung flexible Bewegungen zu ermöglichen, insbesondere verschiedenste Trajektorien beschreiben zu können ist erfindungsgemäß vorgesehen, dass die Röntgeneinrichtung derart ausgestaltet ist, dass die Lage der Propellerachse (z₄) in allen räumlichen Richtungen (z_{1,} z₂, z₃) veränderbar ist.

## Beschreibung

Die Erfindung betrifft eine Röntgeneinrichtung mit einem eine Röntgenquelle und einen Röntgendetektor tragenden C-Bogen und mit einer den C-Bogen an einem Gelenk tragenden Aufhängevorrichtung, wobei Röntgenquelle und Röntgendetektor um eine durch das Gelenk verlaufende Propellerachse rotierbar sind.

Derartige Röntgeneinrichtungen sind allgemein bekannt und werden vielfach zur Bildgebung für die medizinische Diagnostik eingesetzt. Dabei befindet sich der zu untersuchende Patient in der Regel in horizontaler Lage auf einem Patiententisch. Zur Erstellung von Röntgenprojektionen, insbesondere für die Gefäßdiagnostik (Angiographie), ist die Projektionsrichtung in gewissen Grenzen veränderbar. So sind Röntgenquelle und Röntgendetektor in einem Winkelbereich um die Längsachse des Patienten herum rotierbar, der Winkel zwischen der Projektionsrichtung und der Längsachse des Patienten ist einstellbar und die den C-Bogen tragende Aufhängevorrichtung ist meist derart im Raum verankert, dass sie in einem Winkelbereich um eine vertikale Achse rotierbar ist. Dadurch kann die Röntgenquelle zeitlich nacheinander verschiedene Positionen einnehmen und bestimmte Trajektorien beschreiben, beispielsweise eine halbkreisförmige Trajektorie, wobei sich die Projektionsrichtung entsprechend ändert. Mittels einer Darstellung der dabei erhaltenen Projektionen in schneller zeitlicher Folge auf einem Bildschirm ergibt sich für einen diagnostizierenden Arzt dann eine bessere Vorstellung der dreidimensionalen Anordnung, z.B. der untersuchten Gefäße. Auch lassen sich dreidimensionale Bilder des untersuchten Volumens aus diesen Projektionen berechnen.

Bei den bekannten Röntgeneinrichtungen sind jedoch die Einstell- und Bewegungsmöglichkeiten der Röntgenquelle aufgrund des mechanischen Aufbaus der Röntgeneinrichtung begrenzt. So lässt sich insbesondere die Lage der sogenannten Propellerachse, d.h. der Achse, welche durch das den C-Bogen mit der Aufhängevorrichtung verbindende Gelenk verläuft, nur in einer horizontalen durch den Patienten verlaufenden Ebene verändern. Aufgrund dieser Begrenzung ist auch die Form der möglichen Trajektorien begrenzt. So ist es bei den bekannten Röntgeneinrichtungen beispielsweise nicht möglich, mit der Röntgenquelle eine Trajektorie in Form eines Vollkreises um den Patienten herum oder zweier zueinander gekippter Vollkreise um den Patienten herum zu beschreiben. Dies liegt zwar einerseits auch daran, dass das den C-Bogen mit der Aufhängevorrichtung verbindende Gelenk nicht geeignet ausgestaltet ist, ist jedoch im wesentlichen darauf zurückzuführen, dass die gesamte Mechanik der Röntgeneinrichtung, insbesondere das Gewicht des C-Bogens, derartige Bewegungen nicht zulässt.

Der Erfindung liegt demnach die Aufgabe zugrunde, eine Röntgeneinrichtung zu schaffen, die mechanisch stabil ist und mit der sich verschiedene Trajektorien realisieren lassen.

Diese Aufgabe wird ausgehend von einer eingangs genannten Röntgeneinrichtung durch eine Röntgeneinrichtung gemäß Anspruch 1 gelöst.

Der Erfindung liegt dabei die Erkenntnis zugrunde, dass bei den bekannten Röntgeneinrichtungen die beschreibbaren Trajektorien auch dadurch begrenzt sind, dass die Lage der Propellerachse nur in einer Ebene veranderbar ist. Erfindungsgemäß soll deshalb die Ausgestaltung der Röntgeneinrichtung, insbesondere des C-Bogens, des Gelenks und der Aufhängevorrichtung, derart erfolgen, dass die Lage der Propellerachse auch in weiteren Richtungen veränderbar ist. Bei geeigneten Ausgestaltungen kann auch die Mechanik des C-Bogens einfacher und vor allem das Gewicht des C-Bogens geringer ausfallen, wodurch sich dann die gewünschten Trajektorien mit der Röntgenröhre realisieren lassen.

Vorteilhafte Weiterbildungen der Erfindung ergeben sich aus den weiteren Ansprüchen.

Besonders vorteilhaft ist eine Weiterbildung. gemäß der die Aufhängevorrichtung und/oder eine die Aufhängevorrichtung tragende Haltevorrichtung kreisbogenförmig ausgestaltet sind. Mit dieser Ausgestaltung lässt es sich einfach realisieren, dass sich alle Rotationsachsen der Röntgeneinrichtung, insbesondere auch die Propellerachse durch das sogenannte Isozentrum, in dem das zu untersuchende Objekt angeordnet ist und durch das auch die Projektionslinien (die Verbindungslinie zwischen Röntgenquelle und Röntgendetektor) verlaufen, schneiden. Vorteilhafterweise weist dazu die Aufhängevorrichtung bzw. die Haltevorrichtung dieselbe Krümmung auf wie der C-Bogen.

Gemäß einer bevorzugten Weiterbildung der Erfindung ist vorgesehen, dass die Aufhängevorrichtung derart ausgestaltet ist, dass der C-Bogen und das Gelenk um eine horizontal verlaufende senkrecht auf der Propellerachse stehende Rotationsachse rotierbar sind. Dadurch lässt sich der C-Bogen im Vergleich zu den bekannten Röntgeneinrichtungen wesentlich einfacher und vor allem leichter ausgestalten, da er nur noch die Röntgenquelle und den Röntgendetektor tragen muss, nicht aber weitere mechanische Elemente, die bei den bekannten Röntgeneinrichtungen die Rotationsbewegung um die Rotationsachse ermöglichen. Diese Funktionalität wird bei dieser Weiterbildung durch die Aufhägevorrichtung erfüllt.

Weiterhin ist vorgesehen, dass das Gelenk als Drehgelenk derart ausgestaltet ist, dass Röntgenquelle und Röntgendetektor beliebig oft um 360° um die Propellerachse rotierbar sind. Dies lässt sich besonders bei der vorgenannten Weiterbildung der Erfindung vorsehen, bei der der C-Bogen besonders leicht ausgestaltet sein kann.

Die Erfindung wird nachfolgend anhand der Zeichnungen näher erläutert. Es zeigen:
- Fig. 1: eine bekannte Röntgeneinrichtung gemäß dem Stand der Technik,
- Fig. 2: eine schematische Darstellung der wesentlichen Elemente der bekannten Röntgeneinrichtung gemäß Fig. 1,
- Fig. 3A-3E: Skizzen verschiedener wünschenswerter Trajektorien,
- Fig. 4A, 4B: eine erste Ausführungsform einer erfindungsgemäßen Röntgeneinrichtung in einer Seitenansicht und in einer Draufsicht,
- Fig. 5: die erste Ausführungsform der erfindungsgemäßen Röntgeneinrichtung in einer anderen Position,
- Fig. 6: eine schematische Darstellung der wesentlichen Elemente einer erfindungsgemäßen Röntgeneinrichtung,
- Fig. 7: eine zweite Ausführungsform einer erfindungsgemäßen Röntgeneinrichtung und
- Fig. 8: eine dritte Ausführungsform einer erfindungsgemäßen Röntgeneinrichtung.

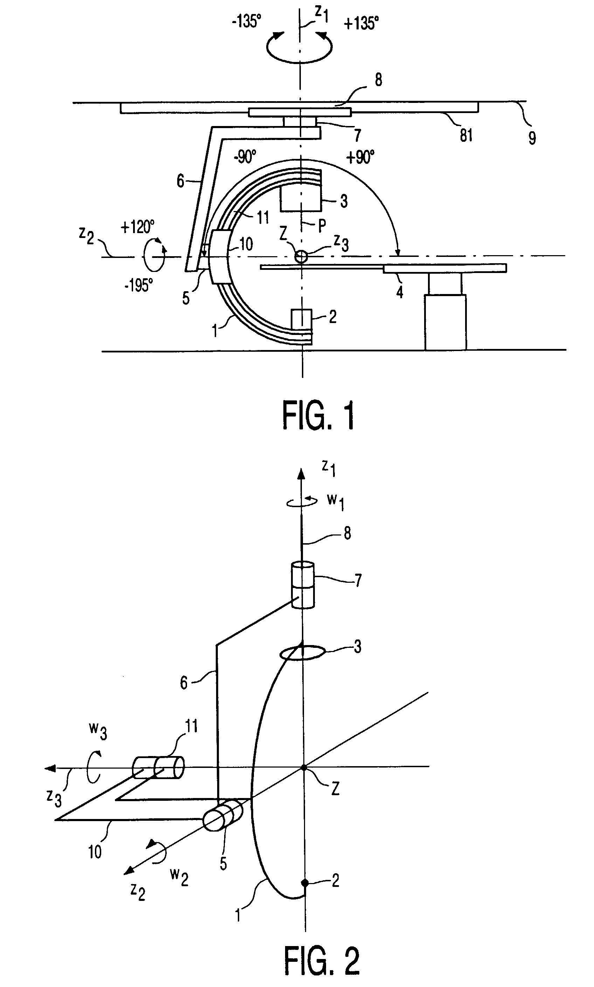

Die in Fig. 1 dargestellte bekannte Röntgeneinrichtung weist als Kernstück einen C-Bogen 1 auf, an dessen Enden eine punktförmige Röntgenquelle 2 bzw. ein flächiger Röntgendetektor 3 angebracht sind. Der C-Bogen 1 wird von einem Gelenk 5, einem sogenannten C-Bogenhalter, gehalten, der seinerseits an einer Aufhängevorrichtung 6, einem sogenannten L-Arm, befestigt ist. Dieser L-Arm 6 wird von einer Deckenhalterung 7, einem sogenannten L-Armhalter, getragen, der wiederum an der Decke 9 oder wie im gezeigten Fall an einem an Deckenschienen 81 hängenden Schlitten 8 befestigt ist. Die Deckenhalterung 7 ist so ausgestaltet, dass der L-Arm 6 manuell um die vertikale Drehachse z₁ in einem Winkelbereich von ± 135° gedreht werden kann. Das Gelenk 5 ist so ausgestaltet, dass der C-Bogen 1 motorisch um die horizontal verlaufende Achse z₂ im Winkelbereich von +120° bis -195° gedreht werden kann. Schließlich kann auch der C-Bogen 1 motorisch um eine Rotationsachse z₃ gedreht werden, die senkrecht auf den beiden genannten Achsen z₁ und z₂ steht, wozu an dem C-Bogen 1 geeignete Mittel, beispielsweise eine an dem Gelenk 5 befestigte Haltevorrichtung 10 mit einem motorisch angetriebenen Zahnrad und eine entsprechende Zahnradschiene 11, vorgesehen sind. Die drei genannten Achsen z₁, z₂, z₃ sind dabei so gewählt, dass sie sich in einem Punkt schneiden, dem sogenannten Isozentrum Z, in dem der zu untersuchende Bereich des auf einem Patiententisch 4 liegenden Patienten angeordnet ist und durch das auch der "mittlere" Röntgenstrahl P von der Röntgenquelle 2 zum Röntgendetektor 3 verläuft. Weitere Bewegungsmöglichkeiten ergeben sich dadurch, dass der Patiententisch 4 in z₂-Richtung verschoben und in der Höhe (in z₁-Richtung) verstellt werden kann und dass die ganze Röntgeneinrichtung an der Schiene 81 in z₂-Richtung verschiebbar ist.

Allgemein ist eine solche C-Bogen-Röntgeneinrichtung mit einer Kette von Armen bzw. "Gliedern" ausgestattet, die durch Drehgelenke miteinander verbunden sind. Das erste Glied der Kette ist mit dem Gebäude fest oder mittels eines Schlittens verschiebbar verbunden. Das letzte Glied der Kette, der C-Bogen, trägt sowohl die Röntgenquelle als auch den Röntgendetektor. Die Drehgelenke können innerhalb gewisser Grenzen und ggf. motorisch und in kontrollierter Weise verstellt werden. Bei derartigen C-Bogen-Röntgeneinrichtungen schneiden sich die Achsen der Drehgelenke in dem genannten Isozentrum, durch das auch der mittlere Röntgenstrahl verläuft, der die Projektionsrichtung beschreibt. Die möglichen Bewegungen des letzten Gliedes der Kette können durch die Trajektorie der Röntgenquelle beschrieben werden. Jeder Punkt auf der Trajektorie entspricht einer möglichen Projektionsrichtung. Die Trajektorien verlaufen alle auf einer Kugeloberfläche, deren Mittelpunkt im Isozentrum liegt. Infolge der Beschränkungen bei den Drehgelenken sind jedoch die möglichen Trajektorien eingeschränkt.

Für die weiteren Betrachtungen ist es hilfreich, die Kette der Glieder und Drehgelenke schematisch darzustellen, was anhand von Fig. 2 für die in Fig. 1 gezeigte bekannte Röntgeneinrichtung geschieht. Mittels des Gelenks 7 (Deckenhalterung) ist der L-Arm 6 in einem Winkelbereich w₁ um die z₁-Achse rotierbar. Mittels des Gelenks 5 (C-Bogen-Halterung) kann der C-Bogen 1 im Winkelbereich w₂ um die z₂- Achse gedreht werden. Mittels des Gelenks 11, das bei der Röntgeneinrichtung gemäß Fig. 1 mit Hilfe von kreisförmig gebogenen Führungsschienen realisiert ist, kann der C-Bogen im Winkelbereich w₃ (typischerweise ca. 180°) um die z₃-Achse gedreht werden.

Bei der bekannten Röntgeneinrichtung kann der Winkel w₃ motorisch und in kontrollierter Weise kontinuierlich verändert werden. Der Winkel w₂ kann ebenfalls motorisch, jedoch nicht kontinuierlich verändert werden. Die Propellerachse, hier die z₂-Achse, um die der C-Bogen 1 mittels des Gelenks 5 rotieren kann, verläuft bauartbedingt stets in horizontaler Richtung.

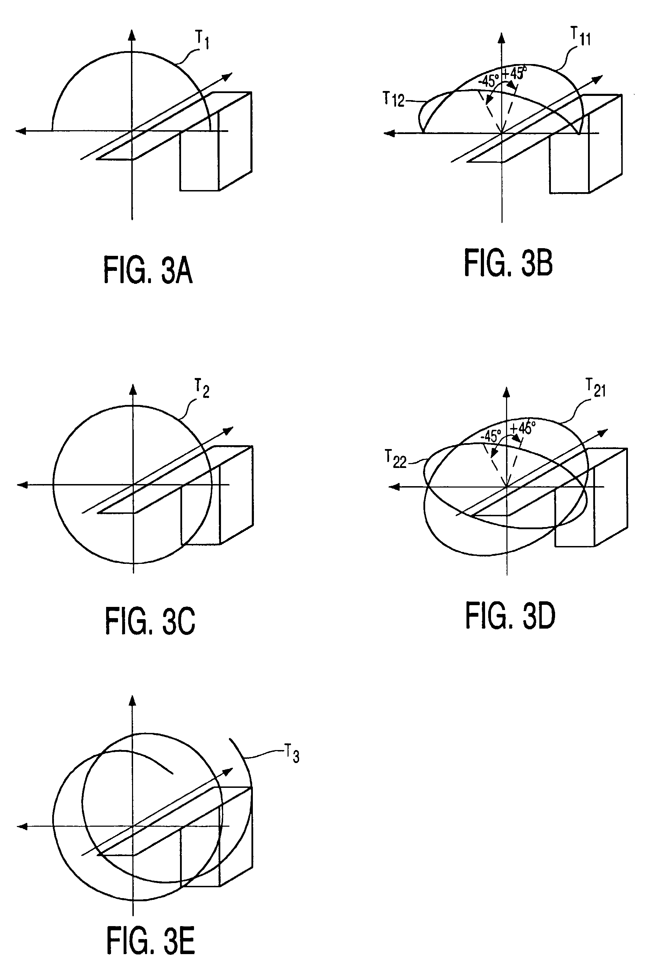

Mit der bekannten Röntgeneinrichtung kann eine in Fig. 3A gezeigte halbkreisförmige Trajektorie T₁ realisiert werden. Allerdings liefert ein solcher Halbkreis nicht genügend Projektionsrichtungen für eine wirklich exakte dreidimensionale Rekonstruktion. Zwar lassen sich auch zwei gekippte und aufeinander senkrecht stehende halbkreisförmige Trajektorien T₁₁, T₁₂ (siehe Fig. 3B) theoretisch realisieren, die auch eine bessere Rekonstruktion ermöglichen würden. Damit genügend Abstand zwischen Patient und Röntgenquelle bzw. Röntgendetektor bleibt, kann in der Praxis jedoch nur eine Verkippung der Halbkreise um den Winkel w₂ = ± 30° realisiert werden. Wünschenswert wären jedoch insbesondere die in den Figuren 3C bis 3E gezeigten Trajektorien in Form eines Vollkreises (Trajektorie T₂), in Form zweier aufeinander senkrecht stehender Vollkreise (Trajektonen T₂₁ und T₂₂) oder in Form einer Helix (Trajektorie T₃).

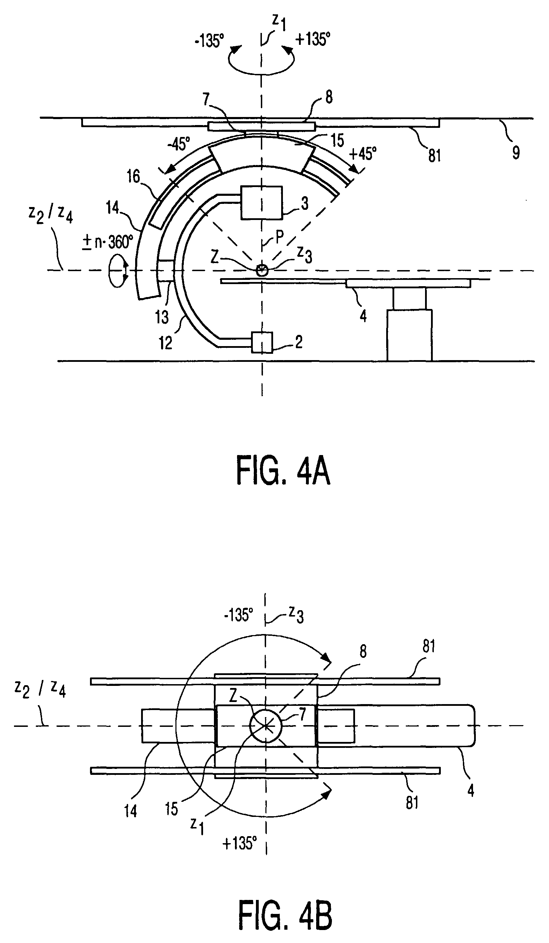

Eine erste Ausführungsform einer erfindungsgemäßen Röntgeneinrichtung ist in den Figuren 4A,4B gezeigt. Dabei trägt die Deckenhalterung 7 eine Haltevorrichtung 15, die wiederum eine Aufhängevorrichtung 14 trägt. Diese Aufhängevorrichtung 14 ist kreisbogenförmig ausgestaltet und ist mit Mitteln 16 ausgestattet, beispielsweise Führungsschienen oder Zahnschienen, die mit entsprechenden Mitteln in der Haltevorrichtung 15 derart zusammenwirken, dass die Aufhängevorrichtung 14 um die z₃- Achse rotierbar ist in einem Winkelbereich von ± 45°. Die Aufhängevorrichtung 14 trägt an ihrem einen Ende ein Drehgelenk 13, an dem wiederum der C-Bogen 12 mit der Röntgenquelle 2 und dem Röntgendetektor 3 angebracht sind.

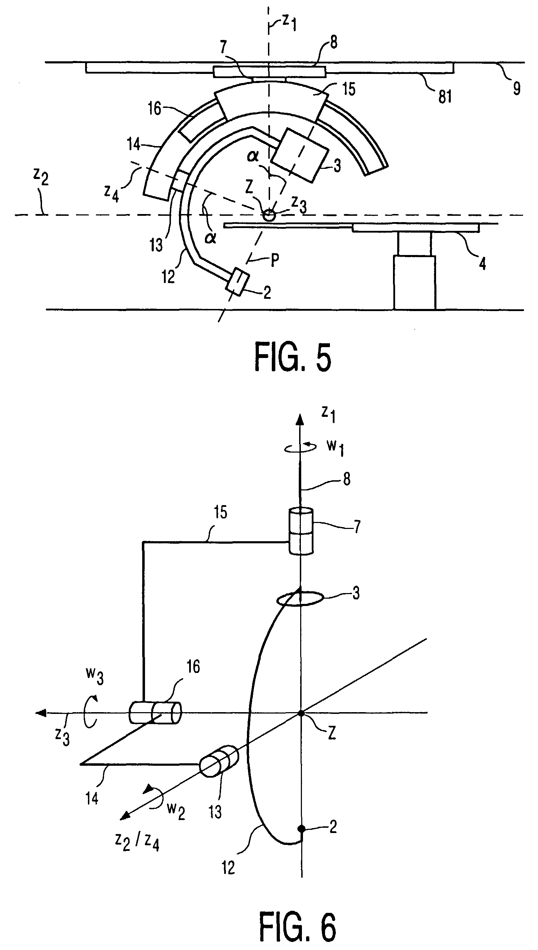

Anders als bei der bekannten Röntgeneinrichtung wird die Drehbewegung um die z₃-Achse nicht durch am C-Bogen angebrachte Mittel erreicht, sondern durch geeignete Mittel an der Aufhängevorrichtung 14. Da an dieser Aufhängevorrichtung 14 mittels des Drehgelenks 13 der C-Bogen angebracht ist, und die Aufhängevorrichtung 14 somit das zweite Glied in der Kette darstellt (und nicht wie bei der bekannten Röntgeneinrichtung das dritte Glied), kann bei der erfindungsgemäßen Röntgeneinrichtung die Lage der Propellerachse, im folgenden als z₄-Achse bezeichnet, in allen drei Raumrichtungen, also auch in z₁-Richtung verändert werden. In der gezeigten Winkelstellung stimmt die Propellerachse z₂ mit der horizontal durch den Patienten verlaufenden Achse z₂ überein. In Fig. 5 ist jedoch eine Winkelstellung gezeigt, in der die Aufhängevorrichtung 14 und somit auch das Drehgelenk 13 und der C-Bogen 12 um einen Winkel α zur z₂-Achse verschwenkt ist. Auch die Projektionsrichtung P ist um denselben Winkel α gegenüber der z₁-Achse verschwenkt.

Eine Draufsicht auf die erfindungsgemäße Röntgeneinrichtung gemäß Fig. 4A ist in Fig. 4B gezeigt, in der insbesondere der Schlitten 8 und die Schienen 81 näher erkennbar sind.

Da der C-Bogen 12 bei der erfindungsgemäßen Röntgeneinrichtung nur noch Röntgenquelle 2 und Röntgendetektor 3 tragen muss, jedoch keine Mittel mehr für eine Drehung des C-Bogens um die z₃-Achse, kann er wesentlich leichter und schlanker ausgestaltet sein als bei der bekannten Röntgeneinrichtung. Es lassen sich deshalb einerseits Drehwinkel von ± 45° um die z₃-Achse realisieren. Andererseits kann auch das Drehgelenk 13 wesentlich einfacher und derart ausgestaltet sein, dass eine Drehung des C-Bogens 12 um die Propellerachse z₄ um 360° und mehr möglich ist. Zur Energieversorgung und Datenübertragung kann das Drehgelenk 13 beispielsweise mit Schleifringen ausgestaltet sein. Bevorzugt ist auch vorgesehen, dass die Drehung um die Propellerachse z₄ motorisch und in kontrollierter Weise kontinuierlich erfolgen kann. Mit der erfindungsgemäßen Röntgeneinrichtung lassen sich somit die in den Figuren 3A bis 3E gezeigten Trajektorien realisieren. Sofern die Drehbewegung in entsprechend kurzer Zeit möglich ist, können auch mit einer geringeren Zahl von Kontrastmitteln Injektionen, ggf. mit einer einzigen Kontrastmittelinjektuion, ausreichend Projektionen für eine dreidimensionale Rekonstruktion ermittelt werden.

Zur Verdeutlichung der Erfindung sind in Fig. 6 nochmals die wesentlichen Elemente der erfindungsgemäßen Röntgeneinrichtung gemäß Fig. 4A schematisch dargestellt. Wie leicht im Vergleich zur Darstellung gemäß Fig. 2 zu erkennen ist, ist die Haltevorrichtung 15 hier mit dem Gelenk 16 verbunden, das die Drehung um die z₃-Achse bewirkt, und das Gelenk 16 ist über die Aufhängevorrichtung 14 mit dem Gelenk 13 verbunden, an dem als letztes Glied der C-Bogen 12 drehbar um die Propellerachse z₄ angebracht ist. Dagegen ist bei der bekannten Röntgeneinrichtung der L-Arm 6 mit dem Gelenk 5 verbunden, an dem auch der C-Bogen 1 angebracht ist, so dass bei einer Drehung um die z₂-Achse nicht nur der C-Bogen sondern auch das Gelenk 11 und der Arm 10 mit bewegt werden müssen.

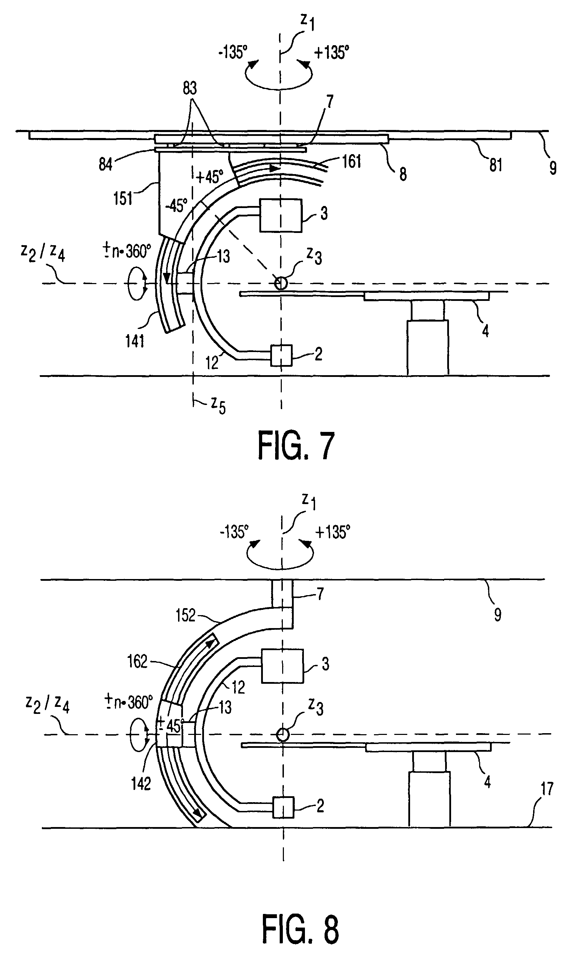

In Fig. 7 ist eine alternative Ausführungsform einer erfindungsgemäßen Röntgeneinrichtung gezeigt. Da bei der in Fig. 4A gezeigten Ausführungsform die Haltevorrichtung 15 stark einseitig belastet ist, ist bei dieser Ausführungsform die Haltevorrichtung 151 an einem Schlitten 84 befestigt, der in Schienen 83 läuft, die kreisbogenförmig um die z₁-Achse verlaufen. Dadurch wird erreicht, dass der Massenschwerpunkt der an der Decke 9 angebrachten Teile der Röntgeneinrichtung auf einer vertikal durch die Haltevorrichtung 151 verlaufenden Schwerpunktachse z₅ liegt, in der auch die Haltevorrichtung mittelbar (über den Schlitten 84) an der Decke 9 befestigt ist. Die Aufhängevorrichtung 141 ist wiederum als Kreisbogensegment ausgestaltet und mit Führungsschienen 161 ausgestaltet, die mit entsprechenden Antriebsmitteln in der Haltevorrichtung 151 zusammenwirken.

Fig. 8 zeigt eine weitere Ausführungsform der erfindungsgemäßen Röntgeneinrichtung, bei der die Haltevorrichtung 152 nahezu als Halbkreisbogensegment ausgestaltet ist und über das Gelenk 7 an der Decke 9 und durch ein nicht gezeigtes Gelenk am Boden 17 befestigt ist. Die Aufhängevorrichtung 142 ist hier als koaxial die Haltevorrichtung 151 umschließendes Bauteil ausgestaltet, das mittels geeigneter Schienen 162 an der Haltevorrichtung 152 entlang bewegt und somit um die z₃-Achse gedreht werden kann.

Die gezeigten Ausführungsformen sind lediglich als Beispiele zur Erläuterung der Erfindung zu verstehen. Es sind noch andere Ausführungsformen denkbar, wobei jedoch wesentlich ist, dass die Lage der Propellerachse in allen räumlichen Richtungen veränderbar ist. Insbesondere kann der C-Bogen auch anders, vor allem leichter ausgestaltet sein. Auch die abgerundete, kreisbogenförmige C-Form ist nicht zwingend, er kann auch eckig ausgestaltet sein.

## Patentansprüche

1. Röntgeneinrichtung mit einem eine Röntgenquelle (2) und einen Röntgendetektor (3) tragenden C-Bogen (12) und mit einer den C-Bogen (12) an einem Gelenk (13) tragenden Aufhängevorrichtung (14), wobei Röntgenquelle (2) und Röntgendetektor (3) um eine durch das Gelenk (13) verlaufende Propellerachse (z₄) rotierbar sind,
dadurch gekennzeichnet,
dass die Röntgeneinrichtung derart ausgestaltet ist, dass die Lage der Propellerachse (z₄) in allen räumlichen Richtungen (z₁, z₂, z₃) veränderbar ist.

2. Röntgeneinrichtung nach Anspruch 1,
dadurch gekennzeichnet,
dass die Aufhängevorrichtung (14) derart ausgestaltet ist, dass der C-Bogen (12) und das Gelenk (13) um eine horizontal verlaufende senkrecht auf der Propellerachse (z₄) stehende Rotationsachse (z₃) rotierbar sind.

3. Röntgeneinrichtung nach Anspruch 1,
dadurch gekennzeichnet,
dass das Gelenk (13) fest an der Aufhängevorrichtung (14) und die Aufhängevorrichtung (14) beweglich an einer Haltevorrichtung (15) angeordnet sind.

4. Röntgeneinrichtung nach Anspruch 3,
dadurch gekennzeichnet,
dass die Aufhängevorrichtung (14) und die Haltevorrichtung (15) koaxial zueinander angeordnet sind.

5. Röntgeneinrichtung nach Anspruch 1 oder 3,
dadurch gekennzeichnet,
dass die Aufhängevorrichtung (14) und/oder die Haltevorrichtung (15) kreisbogenförmig ausgestaltet ist.

6. Röntgeneinrichtung nach Anspruch 5,
dadurch gekennzeichnet,
dass die Aufhängevorrichtung (14) und/oder die Haltevorrichtung (15) dieselbe Krümmung aufweist wie der C-Bogen (12).

7. Röntgeneinrichtung nach Anspruch 1,
dadurch gekennzeichnet,
dass die Aufhängevorrichtung (141) derart an einer Haltevorrichtung (151) angeordnet ist, dass der Massenschwerpunkt der Aufhängevorrichtung (141) und des C-Bogens (12) etwa auf einer vertikalen durch die Haltevorrichtung (151) verlaufende Schwerpunktachse (z₅) liegt.

8. Röntgeneinrichtung nach Anspruch 1,
dadurch gekennzeichnet,
dass das Gelenk (13) als Drehgelenk derart ausgestaltet ist, dass Röntgenquelle (2) und Röntgendetektor (3) beliebig oft um 360° um die Propellerachse (z₄) rotierbar sind.

9. Röntgeneinrichtung nach Anspruch 1,
dadurch gekennzeichnet,
dass das Gelenk (13) derart ausgestaltet ist, dass die Rotationsbewegung von Röntgenquelle (2) und Röntgendetektor (3) motorisch und schrittweise steuerbar ist.

10. Röntgeneinrichtung nach Anspruch 1,
dadurch gekennzeichnet,
dass die Aufhängevorrichtung (14) mittels einer Haltevorrichtung (15) im Raum (9, 17) um eine vertikale Drehachse (z₁) rotierbar angebracht ist.
